(19) Europäisches Patentamt
European Patent Office
Office européen des brevets

(11) Veröffentlichungsnummer: **0 195 329**
**B1**

# (12) EUROPÄISCHE PATENTSCHRIFT

(45) Veröffentlichungstag der Patentschrift: **08.02.89**

(51) Int. Cl.⁴: **C 07 D 249/08**

(21) Anmeldenummer: **86103077.3**

(22) Anmeldetag: **07.03.86**

(54) **Verfahren zur Herstellung des (-)-Antipoden des (E)-1-Cyclohexyl-4,4-dimethyl-3-hydroxy-2-(1,2,4-triazol-1-yl)-pent-1-ens.**

(30) Priorität: **19.03.85 DE 3509823**

(43) Veröffentlichungstag der Anmeldung: **24.09.86 Patentblatt 86/39**

(45) Bekanntmachung des Hinweises auf die Patenterteilung: **08.02.89 Patentblatt 89/6**

(84) Benannte Vertragsstaaten: **AT BE CH DE FR GB IT LI NL**

(56) Entgegenhaltungen:
**WO-A-84/03885**
**DE-A- 3 102 588**
**DE-A- 3 208 142**
**DE-A- 3 221 700**
**DE-A- 3 235 050**
**DE-A- 3 302 120**

**CHEMICAL ABSTRACTS, Band 94, no. 17, 27. April 1981, Columbus, Ohio, USA, BAYER AG, "Vinyltriazoles", p. 767, Zusammenfassung Nr. 139 814c**
**Chem. Pharm. Bull., 31, 837 (1983)**

(73) Patentinhaber: **BAYER AG, Konzernverwaltung RP Patentabteilung, D-5090 Leverkusen 1 Bayerwerk (DE)**

(72) Erfinder: **Kraatz, Udo, Dr., Andreasstrasse 22a, D-5090 Leverkusen (DE)**
Erfinder: **Feyen, Peter, Dr., Mozartstrasse 1, D-4020 Mettmann (DE)**

**EP 0 195 329 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung des bekannten (–)-Antipoden *) des (E)-1-Cyclohexyl-4,4-dimethyl-3-hydroxy-2-(1,2,4-triazol-1-yl)-pent-1-ens.

Es ist bereits bekannt geworden, den (–)-Antipoden des (E)-1-Cyclohexyl-4,4-dimethyl-3-hydroxy-2-(1,2,4-triazol-1-yl)-pent-1-ens dadurch herzustellen, dass man die entsprechende racemische Verbindung mit einem optisch aktiven Säurechlorid umsetzt, das entstehende Ester-Diastereomeren-Gemisch auf chromatographischem Wege trennt und den Ester, der den (–)-Antipoden enthält, verseift (vgl. DE-OS 3 302 122). Nachteilig an diesem Verfahren ist aber, dass es nur zur Synthese geringer Mengen des gewünschten Antipoden geeignet ist.

Weiterhin ist bereits bekannt geworden, dass man Ketone mit Reduktionsmitteln in Gegenwart verschiedener chiraler Hilfsreagentien zu optisch aktiven Carbinolen reduzieren kann (vgl. Chem. Pharm. Bull. 31, 837 (1983) und EP-OS 0 054 431). Unbefriedigend ist jedoch, dass dieses Verfahren nicht generell anwendbar ist. So lassen sich Ketone, die keine aromatischen Gruppen enthalten, nur in Carbinole mit einer für praktische Zwecke nicht ausreichenden optischen Reinheit überführen.

Es wurde nun gefunden, dass man den (–)-Antipoden des (E)-1-Cyclohexyl-4,4-dimethyl-3-hydroxy-2-(1,2,4-triazol-1-yl)-pent-1-ens der Formel

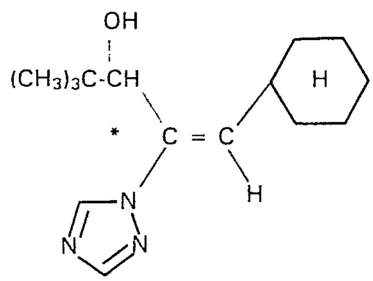

(I)

erhält, indem man (E)-Isomeres des 1-Cyclohexyl-4,4-dimethyl-2-(1,2,4-triazol-1-yl)-pent-1-en-3-ons der Formel

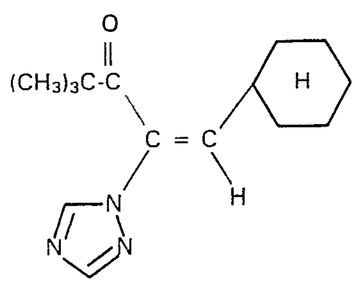

(II)

mit Lithiumaluminium-hydrid in Gegenwart eines inerten organischen Verdünnungsmittels sowie in Gegenwart von (–)-Antipoden des N-Methyl-ephedrins und gegebenenfalls in Gegenwart von N-Ethylanilin bei Temperaturen zwischen –70°C und +50°C umsetzt.

Es ist als äusserst überraschend zu bezeichnen, dass sich der (–)-Antipode des (E)-1-Cyclohexyl-4,4-dimethyl-3-hydroxy-2-(1,2,4-triazol-1-yl)-pent-1-ens der Formel (I) nach dem erfindungsgemässen Verfahren in sehr hoher Ausbeute und ausgezeichneter Reinheit darstellen lässt, denn aufgrund des bekannten Standes der Technik konnte nicht damit gerechnet werden, dass die Umsetzung selektiv zu dem gewünschten Produkt führt. Vor allen Dingen war nicht zu erwarten, dass die Umsetzung auch bei relativ hohen Temperaturen nahezu frei von Nebenreaktionen abläuft.

Das erfindungsgemässe Verfahren zeichnet sich durch eine Reihe von Vorteilen aus. So sind die Reaktionskomponenten auch in grösseren Mengen zugänglich und auch im technischen Massstab problemlos zu handhaben. Ferner ist der zur Durchführung des Verfahrens erforderliche apparative Aufwand gering, und die Aufarbeitung des nach beendeter Umsetzung anfallenden Reaktionsgemisches bereitet keine Schwierigkeiten. Insbesondere jedoch lässt sich der (–)-Antipode des (E)-1-Cyclohexyl-4,4-dimethyl-3-hydroxy-2-(1,2,4-triazol-1-yl)-pent-1-ens der Formel (I) nach dem erfindungsgemässen Verfahren in höherer Ausbeute und besserer optischer Reinheit herstellen als nach der bisher bekannten Methode, bei der eine klassische Racematspaltung vorgenommen wird.

Der nach dem erfindungsgemässen Verfahren herstellbare (–)-Antipode des (E)-1-Cyclohexyl-4,4-dimethyl-3-hydroxy-2-(1,2,4-triazol-1-yl)-pent-1-ens ist durch die Formel (I) eindeutig charakterisiert. In dieser Formel ist das asymmetrisch substituierte Kohlenstoffatom, welches das Chiralitätszentrum darstellt, durch ein (*) markiert. Durch den Buchstaben «E» vor dem systematischen Namen der Verbindung der Formel (I) wird ausgedrückt, dass der Cyclohexyl-Rest und der 1,2,4-Triazolyl-Rest auf *entgegengesetzten* Seiten der Doppelbindung stehen.

Verwendet man Lithiumaluminiumhydrid als Reduktionsmittel, (–)-Antipoden des N-Methylenephedrins als chirales Hilfsreagenz und N-Ethyl-anilin als zusätzliches Amin, so kann der Verlauf des erfindungsgemässen Verfahrens durch das folgende Formelschema wiedergegeben werden:

*) Unter dem (–)-Antipoden ist hier jeweils dasjenige Enantiomere zu verstehen, das die Schwingungsebene von linear polarisiertem Licht der Natrium-D-Linie nach links dreht.

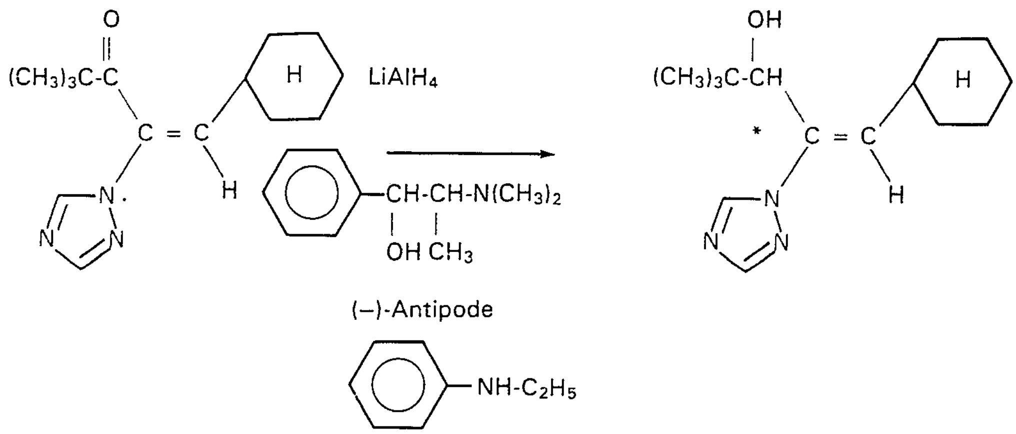


Das bei der Durchführung des erfindungsgemässen Verfahrens als Ausgangsmaterial benötigte E-Isomere des 1-Cyclohexyl-4,4-dimethyl-2-(1,2,4--triazol-1-yl)-pent-1-en-3-ons der Formel (II) ist bereits bekannt (vgl. DE-OS 3 322 818).

Bei der Durchführung des erfindungsgemässen Verfahrens fungiert Lithiumaluminium-hydrid als Reduktionsmittel.

Als Verdünnungsmittel können bei der erfindungsgemässen Umsetzung alle für derartige Reaktionen üblichen inerten organischen Solventien eingesetzt werden. Vorzugsweise in Betracht kommen Ether, wie Diethylether, Tetrahydrofuran und tert.-Butyl-methyl-ether.

Die Reaktionstemperaturen können bei der Durchführung des erfindungsgemässen Verfahrens innerhalb eines bestimmten Bereiches variiert werden. Man arbeitet bei Temperaturen zwischen −70°C und +50°C, vorzugsweise zwischen −20°C und +40°C.

Das erfindungsgemässe Verfahren wird im allgemeinen unter Normaldruck durchgeführt.

Vorzugsweise arbeitet man unter Schutzgasatmosphäre. Als Schutzgase können dabei alle üblichen unter den Reaktionsbedingungen inerten Gase eingesetzt werden. Vorzugsweise verwendbar sind Stickstoff und Argon.

Bei der Durchführung des erfindungsgemässen Verfahrens setzt man auf 1 Mol an (E)-Isomerem des 1-Cyclohexyl-4,4-dimethyl-2-(1,2,4-triazol-1-yl)--pent-1-en-3-ons der Formel (II) im allgemeinen 1 bis 3 Mol, vorzugsweise 1,3 bis 2 Mol, Lithiumaluminium-hydrid und 1 bis 3 Mol, vorzugsweise 1,3 bis 2 Mol, an (−)-Antipoden des N-Methyl-ephedrins sowie gegebenenfalls 2 bis 4 Mol, vorzugsweise 2,5 bis 3,5 Mol, an N-Ethyl-anilin ein.

Im allgemeinen geht man bei der Durchführung des erfindungsgemässen Verfahrens so vor, dass man den (−)-Antipoden des N-Methyl-ephedrins, gegebenenfalls in einem organischen Verdünnungsmittel gelöst, bei Temperaturen zwischen 0°C und 50°C, vorzugsweise zwischen 10°C und 40°C, in eine Suspension von Lithiumaluminium-hydrid in einem organischen Verdünnungsmittel eintropft, anschliessend gegebenenfalls N-Ethyl-anilin zugibt und danach bei Temperaturen zwischen −70°C und 0°C, vorzugsweise zwischen −20°C und −10°C, eine Lösung von (E)-Isomerem des 1-Cyclohexyl-4,4-dimethyl-2-(1,2,4-triazol-1-yl)-pent-1-en-3-ons der Formel (II) in einem organischen Verdünnungsmittel zutropft. Die Aufarbeitung erfolgt nach üblichen Methoden. Im allgemeinen verfährt man in der Weise, dass man Wasser hinzufügt, das Reaktionsgemisch ansäuert, die organische Phase abtrennt, die wässrige Phase mehrfach mit einem mit Wasser wenig mischbaren organischen Solvens extrahiert, die vereinigten organischen Phasen mit Wasser wäscht und danach einengt. Der verbleibende Rückstand kann durch Digerieren mit geeigneten organischen Solventien oder durch Umkristallisation weiter gereinigt werden.

Der nach dem erfindungsgemässen Verfahren herstellbare (−)-Antipode des (E)-1-Cyclohexyl-4,4-dimethyl-3-hydroxy-2-(1,2,4-triazol-1-yl)-pent-1-ens der Formel (I) und dessen Verwendung zur Regulierung des Pflanzenwachstums sind bekannt (vgl. DE-OS 3 302 122).

Die Durchführung des erfindungsgemässen Verfahrens wird durch das folgende Beispiel veranschaulicht.

*Herstellungsbeispiel*

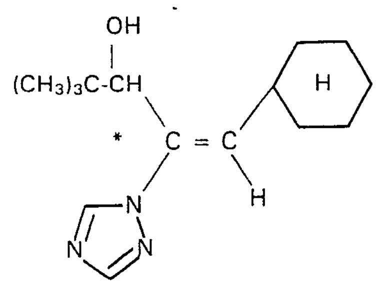

(I)

Eine Lösung von 628 g (3,5 Mol) des (−)-Antipoden des N-Methyl-ephedrins in 7,5 Liter tert.-Butyl-methylether wird bei 20°C unter Argonatmosphäre innerhalb von 45 Minuten in eine Suspension von 133 g (3,5 Mol) Lithiumaluminiumhydrid in 1,5 Liter tert.-Butyl-methylether eingetropft. Es wird 30 Minuten bei 40°C nachgerührt, dann auf 10°C abgekühlt und innerhalb von 45 Minuten mit 848 g (7 Mol) N-Ethyl-anilin versetzt. Man rührt 1 Stunde bei 40°C nach, kühlt dann auf −15°C ab und tropft innerhalb von 30 Minuten eine Lösung von 522 g (2 Mol) des (E)-Isomeren des 1-Cyclohexyl-4,4-dimethyl-2--(1,2,4-triazol-1-yl)-pent-1-en-3-ons in 4,7 Liter

tert.-Butyl-methylether hinzu, wobei die Temperatur des Reaktionsgemisches auf −10°C bis −15°C gehalten wird. Man rührt weitere 16 Stunden bei −15°C und gibt dann langsam 8 Liter Wasser hinzu. Das resultierende Reaktionsgemisch wird mit 3,5 Litern 10%iger wässriger Salzsäure versetzt. Anschliessend wird die organische Phase abgetrennt, und die wässrige Phase wird dreimal mit je 2,5 Liter tert.-Butyl-methylether extrahiert. Die vereinigten organischen Phasen werden zweimal mit je 2 Liter Wasser gewaschen und dann bei einem Druck von 20 mbar eingedampft. Der verbleibende kristalline Rückstand wird mit Cyclohexan verrührt, abgesaugt und unter vermindertem Druck bei 35°C getrocknet. Man erhält auf diese Weise 466 g (87% der Theorie) des (−)-Antipoden des (E)-1-Cyclohexyl-4,4-dimethyl-3-hydroxy-2-(1,2,4-triazol-1-yl)-pent-1-ens in Form eines kristallinen Produktes.

Schmelzpunkt: 162-164°C.

$[\alpha]_D^{20} = -81{,}4°$ (C = 67 mg/10 ml $CHCl_3$)

Das Produkt besitzt eine optische Reinheit von 100%.

## Patentansprüche

1. Verfahren zur Herstellung des (−)-Antipoden des (E)-1-Cyclohexyl-4,4-dimethyl-3-hydroxy-2-(1,2,4-triazol-1-yl)-pent-1-ens der Formel

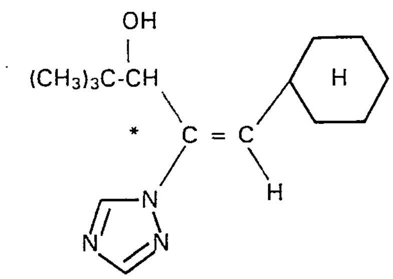

(I)

dadurch gekennzeichnet, dass man (E)-Isomeres des 1-Cyclohexyl-4,4-dimethyl-2-(1,2,4-triazol-1-yl)-pent-1-en-3-ons der Formel

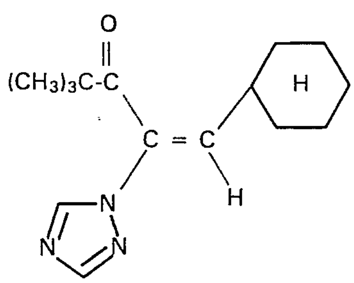

(II)

mit Lithiumaluminium-hydrid in Gegenwart eines inerten organischen Verdünnungsmittels sowie in Gegenwart von (−)-Antipoden des N-methyl-ephedrins und gegebenenfalls in Gegenwart von N-Ethyl-anilin bei Temperaturen zwischen −70°C und +50°C umsetzt.

2. Verfahren gemäss Anspruch 1, dadurch gekennzeichnet, dass man als inerte organische Verdünnungsmittel Diethylether, Tetrahydrofuran oder tert.-Butyl-methyl-ether einsetzt.

3. Verfahren gemäss Anspruch 1, dadurch gekennzeichnet, dass man die Umsetzung bei Temperaturen zwischen −20°C und +40°C durchführt.

4. Verfahren gemäss Anspruch 1, dadurch gekennzeichnet, dass man auf 1 Mol an (E)-Isomerem des 1-Cyclohexyl-4,4-dimethyl-2-(1,2,4-triazol-1-yl)-pent-1-en-3-ons der Formel (II) 1 bis 3 Mol Lithiumaluminium-hydrid und 1 bis 3 Mol an (−)-Antipodem des N-Methyl-ephedrins sowie gegebenenfalls 2 bis 4 Mol an N-Ethylanilin einsetzt.

## Claims

1. Process for the preparation of the (−)-antipode of (E)-1-cyclohexyl-4,4-dimethyl-3-hydroxy-2-(1,2,4-triazol-1-yl)-pent-1-ene of the formula

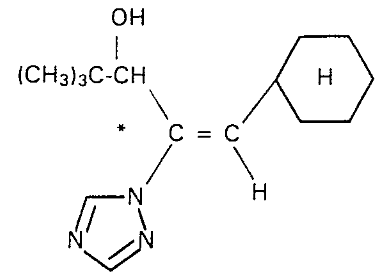

(I)

characterised in that the (E)-isomer of 1-cyclohexyl-4,4dimethyl-2-(1,2,4-triazol-1-yl)-pent-1-en-3-one of the formula

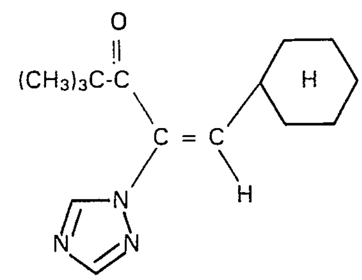

(II)

is reacted with lithium aluminium hydride in the presence of an inert organic diluent and in the presence of (−)-antipodes of N-methyl-ephedrine and , if appropriate, in the presence of N-ethyl-aniline, at temperatures between −70°C and +50°C.

2. Process according to Claim 1, characterised in that the inert organic diluent employed is diethyl ether, tetrahydrofuran or tert.-butyl methyl ether.

3. Process according to Claim 1, characterised in that the reaction is carried out at temperatures between −20°C and +40°C.

4. Process according to Claim 1, characterised in that 1 to 3 mol of lithium aluminium hydride and 1 to 3 mol of the (−)-antipode of N-methyl-ephedrine and, if appropriate, 2 to 4 mol of N-ethyl-aniline are employed per mol of the (E)-isomer of 1-cyclohexyl-4,4-dimethyl-2-(1,2,4-triazol-1-yl)-pent-1-en-3-one of the formula (II).

## Revendications

1. Procédé pour la fabrication de l'antipode (−) du (E)-1-cyclohexyl-4,4-dimethyl-3-hydroxy-2-(1,2,4-triazole-1-yl)-1-pentène de formule (I)

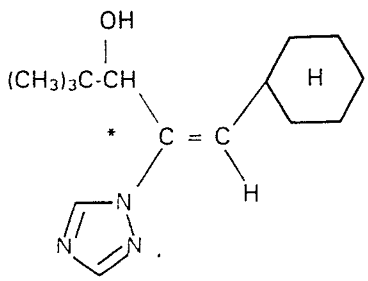

(I)

caractérisé en ce que l'on fait réagir l'isomère (E) de la 1-cyclohexyl-4,4-diméthyl-2-(1,2,4-triazole-1-yl)-1-pentène-3-one de formule (II)

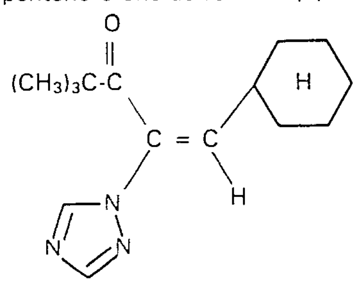

(II)

avec l'hydrure de lithiumaluminium à des températures comprises entre −70 et + 50°C en présence d'un agent diluant organique inerte et en présence d'antipode (−) de la N-méthyléphédrine et éventuellement en présence de N-éthylaniline.

2. Procédé selon la revendication 1, caractérisé en ce que l'on utilise comme agent diluant organique inerte l'éther diéthylique, le tétrahydrofuranne ou l'éther tert-butyl-méthylique.

3. Procédé selon la revendication 1, caractérisé en ce que l'on met en œuvre la réaction à des températures comprises entre −20 et +40°C.

4. Procédé selon la revendication 1, caractérisé en ce que l'on utilise 1 à 3 moles d'hydrure de lithiumaluminium et 1 à 3 moles d'antipode (−) de la N-méthyléphédrine et éventuellement 2 à 4 moles de N-éthylaniline pour 1 mole d'isomère (E) de la 1-cyclohexyl-4,4-diméthyl-2-(1,2,4-triazole-1-yl)-1-pentène-3-one de formule (II).